# EUROPEAN PATENT APPLICATION

(11) **EP 1 992 355 A1**
(43) Date of publication of application: **19.11.2008**
(21) Application number: 07009827.2
(22) Date of filing: 16.05.2007
(51) Int. Cl.: A61K 38/18, A61K 45/06, A61P 39/00, A61P 29/00

(54) **Pharmaceutical combination medication comprising NSAIDs or cytoxic drugs**

(71) Applicant: Spanuth, Eberhardt, 69221 Dossenheim (DE); Kötting, Jochem, 77963 Schwanau (DE)
(72) Inventor: Spanuth, Eberhardt, 69221 Dossenheim (DE); Kötting, Jochem, 77963 Schwanau (DE)
(74) Representative: Oser, Andreas

(57) **Abstract**

A pharmaceutical combination preparation comprises as active agents, in a common or in separate pharmaceutically acceptable formulation(s), a drug selected from the group of COX-1 and/or COX-2 inhibitors; and erythropoietin (EPO) or an analogue of EPO. There are also described combination medications involving the use of cytotoxic drugs and EPO or an analogue of EPO.

## Description

The present invention relates to a new concept of pharmaceutical combination preparation involving the use or administration of non-steroidal anti-inflammatory drugs (NSAIDs), in particular COX-1 inhibitors and/or COX-2 inhibitors, or other drugs belonging to the group of cytotoxic therapeutic agents having adverse vascular side effects and especially cardiotoxicity.

Drugs for relief of inflammatory pain such as NSAIDs, which include COX-1 and COX-2 inhibitors, are known to be associated with side effects. In an attempt to develop NSAIDs without the side effect of gastro-intestinal bleeding, COX-2 specific inhibitors were introduced as agents for relief of inflammatory pain associated with, for example, rheumatic diseases, chronic polyarthritis, osteoarthritis and rheumatoid arthritis. However, therapy with COX-2 inhibitors has been found to be associated with an increased risk of thrombotic and especially cardio-vascular events (e.g. Topol in N. Engl. J. Med. 2004, 351, pp. 1707-1709; Bombardier et al., N. Engl. J. Med. 2000, 323, pp. 1520-1528). Studies such as the socalled APPROVe study have established a higher risk of cardiovascular events after 18 months. Since similar data have been obtained with different COX-2 inhibitors (rofecoxib and coxiben), an increased prothrombotic activity and thus an increased cardio-vascular risk can be assumed for classes of drug compounds displaying cardio-toxic and cardiovascular side effects (concerning COX-2 inhibitors, see Mukherjee et al. in JAMA 2001, 286, pp. 195-199; and G.A. FitzGerald in N. Engl. J. Med. 2004, 351, pp. 1709-1711).

However, even with the well-known anti-platelet and antithrombic agent aspirin, which had been demonstrated to display true cardiovascular protective effect among a large variety of other agents whose primary mode of action on the vascular system was through inhibition of platelet aggregation or adhesion, or both (see "Antiplatelet Trialists' Collaboration", BMJ 1994, 308, pp. 81-106), randomized trials including a comparison between users of both, aspirin and COX2 inhibitors, and users of COX-2 inhibitors alone (i.e. nonusers of aspirin) did not show significant heterogeneity in statistically controlled trials of the rate ratios for vascular events as well as for myocardial infarction, stroke, and vascular death (see P.M. Kearney et al. in BMJ 2006, 332, pp. 1302-1308). Hence, the prophylactic and/or therapeutic use of COX inhibitors, especially COX-2-specific inhibitors, is still hampered.

The present invention enables a basically new concept based on vascular-protective effects of erythropoeitin (EPO) or analogues of EPO for patients undergoing, or are prescribed to undergo, prophylaxis and/or treatment of NSAIDs of type COX-1 and/or COX-2 inhibitors, especially specific COX-2 inhibitors. In surprising contrast to previously ineffective concurrent use of the most common anti-platelet/anti-thombotic drug, aspirin, with COX-2 specific drugs (see P.M. Kearney et al., cit. loc.), the concurrent use of EPO or EPO analogue has been devised to be tailored for counter-matching the vasculardamaging side effects associated with COX-1 and/or COX-2 inhibitors. Thus, the pharmaceutical combination preparation or combination medication of the present invention can establish a balanced counter-action against a drug selected from the group of COX-1 and/or COX-2 inhibitors, by using EPO or an analogue thereof to enhance vascular repairing processes and new angiogenesis, thereby displaying appropriate endothel, nephro- and cardio-protective effects. While EPO and its analogues generally have widespread and complex functions, including pleiotropic effects and erythropoiesis and even increased formation of thrombocytes, their stimulating effect on circulating endothelial progenitor cells has been identified to play the critical role for effectively counteracting the adverse effects of COX-1 and/or COX-2 inhibitors or cytotoxic drugs according to the present invention.

In one embodiment, the present invention provides a pharmaceutical combination preparation comprising as active agents, in a common or in separate pharmaceutically acceptable formulation(s), a drug selected from the group of COX-1 and/or COX-2 inhibitors; and erythropoietin (EPO) or an analogue of EPO. Usually, the combination preparation further comprises a pharmaceutically acceptable carrier or excipient, or other additives. The pharmaceutical combination preparation according to this embodiment is particularly suitable for separate, simultaneous or sequential use in the prophylaxis and/or therapy of inflammation, especially to alleviate pain associated with inflammation.

In another embodiment, the present invention provides a pharmaceutical combination medication for separate, simultaneous or sequential use in prophylaxis and/or treatment of inflammation, said combined medication comprising as active agents, in a common or in separate pharmaceutically acceptable formulation(s), a drug selected from the group of COX-1 and/or COX-2 inhibitors; and erythropoietin (EPO) or an analogue of EPO. Usually, the combination preparation further comprises a pharmaceutically acceptable carrier or excipient, or other additives.

In a preferred embodiment, erythropoietin (EPO) or an analogue of EPO is used in an amount effective to stimulate or to increase the number of endothelial progenitor cells. A suitable test to determine stimulation and increase of endothelial progenitor cells is described for example by F.H. Bahlmann et al. in Kidney International 2003, 64, pp. 1648-1652; F.H. Bahlmann et al. in Blood 2004, 109, pp. 921-925; and F.H. Bahlmann et al. in Circulation 2004, 110, pp. 1006-1012.

The drug selected from the group of COX-1 and/or COX-2 inhibitors is contained in an amount therapeutically effective to treat the desired disease. The disease may be inflammation, in particular arthritis, or other COX-1 and/or COX-2 inhibitor treatable diseases such as cancer.

The present invention can also be applied to other treatment concepts, by using EPO or its analogues in a combination medication with other drugs which potentially have cardiotoxic or other adverse vascular side effects, such as cytotoxic and cardiotoxic drugs. According to the present invention, such an alternative embodiment of a combination medication, or of a method for controlling or for reducing the risk of cardio-toxic or adverse vascular effects associated with the therapeutic administration of a cytotoxic or cardiotoxic drug, comprises a combination of a cytotoxic drug for administration to a patient in need of such therapeutic treatment and EPO or an analogue thereof for separate, simultaneous or subsequent administration to said patient. Hence, the present invention further provides a method for adjuvant treatment and/or adjuvant prophylaxis in diseases treated by cytotoxic or cardiotoxic drug therapy, including colon adenoma, cancer and adenomatus polyps. The cytotoxic or cardiotoxic drug is a chemotherapeutic drug and in particular an anti-cancer drug having cardio-toxic or adverse vascular side effects, thus causing diseases such as kidney diseases, peripheral arterial disease, congestive heart failure, myocarditis, cardiomyopathy, and cerebral vascular disturbances.

Cytotoxic or cardiotoxic drug examples are such as cytostatic agents such as Vinblastin, Vincristine, Vinorelbin, Vindesine, Paclitaxel (Taxol) and Docetaxel ; alkylating agents such as Chlorambucil, Chlormethine, Cyclophosphamide, Ifosfamide, Melphalan, Carmustine, Fotemustine, Lomustine, Streptozocin, Carboplatin, Cisplatin, Oxaliplatin, BBR3464, Busulfan, Dacarbazine, Mechlorethamine, Procarbazine, Temozolomide, ThioTEPA, Uramustine; anthracycline such as Doxorubicin, Daunorubicin, Epirubicin, Idarubicin, Valrubicin and Mitoxantrone; antimetabolites such as Aminopterin, Methotrexate, Pemetrexed, Raltitrexed, Cladribine, Clofarabine, Fludarabine, Mercaptopurine, Pentostatin, Thioguanine, Capecitabine, Cytarabine, Fluorouracil, Floxuridine, Gemcitabine; sulfonamides such as sulfonamide, sulfonureas and thiazide; antibiotics such as Bleomycin, Hydroxyurea, Mitomycin, and Actinomycin; antimycotics such as Itraconazole, Ketoconazole, Bupivacaine; angiogenese- and neoangiogenese-inhibitors such as Anti-Invasive Factor (AIF), retinoic acids, paclitaxel (taxol), suramin, Tissue Inhibitor of metalloproteinase- (TIMP), Plasminogen Activator Inhibitor-1 (PA), Lobenzarid disodium, Thalidomide, angiostatic steroid, metalloproteinase inhibitors (e.g. BB94), in particular anti-angiogenic factores showing positive inhibition in the Chick Chorioallantoic Membrane (CAM) assay; monoclonal antibodies such as Alemtuzumab, Bevacizumab, Cetuximab, Gemtuzumab, Panitumumab, Rituximab, Tositumomab and Trastuzumab; antipsychotics such as Clozapine, Risperidone, Haloperidol, Olanzapine, Quetiapine; photosensitizers such as aminolevulinic acid, methyl aminolevulinate, Porfimer sodium and Verteporfin; catecholamines such as epinephrine, norepinephrine and dopamine; and other cytotoxic or cardiotoxic drugs, respectively having cytotoxic or cardiotoxic properties.

These other treatment concepts are valuable in that cardiotoxic or adverse vascular effects of cytotoxic or cardiotoxic drugs can be treatedthemselves and directly, different or independent from the purpose of conventionally using EPO for erythropoiesis in adjuvant cancer therapy or in case of renal therapy or even renal failure. In line with this alternative new concept, EPO or its analogue should therefore be used in an amount sufficient to activate, or increase the number of endothelial progenitor cells, but should preferably be limited to a low dose which means a dose level which does not increase the hematocrite level. As mentioned, suitable test to determine stimulation and increase of endothelial progenitor cells is described for example by F.H. Bahlmann et al. in Kidney International 2003, 64, pp. 1648-1652; F.H. Bahlmann et al. in Blood 2004, 109, pp. 921-925; and F.H. Bahlmann et al. in Circulation 2004, 110, pp. 1006-1012). Thus, the amounts of EPO or its analogue that can be preferably used according to the present invention can be controlled to be lower than conventionally used for establishing and mantaining an erythropoiesis status.

Preferably, the dose applied to a patient is lower than about 1.0 pg/kg bodyweight, more preferably lower than 0.5 pg/kg bodyweight, and in particular lower than 0.2 pg/kg bodyweight. The combination medication correspondingly comprises EPO or its analogue at a low dose, e.g. at an amount of lower than 100 pg, preferably lower than 50 pg, particularly lower than 10 pg, with respect to the total amount of the common or separate EPO or EPO analogue containing formulation (1000 I.E. typically corresponds to about 8.3-8.4 pg active agent EPO). The treatment scheme of such a low dose use beneficially includes administration of one dose per day, up to one dose per week. In particular, one or two low doses per week are sufficiently effective to activate, or increase the number of endothelial progenitor cells.

The possible alternatives of using separately, simulataneously or subsequently both, the drug selected from the group of COX1 and/or COX-2 inhibitors and EPO or an analogue thereof, means that at any time during prophylaxis and therapy, the selected drug and EPO or its analogue may exert their respective action both within the treated patient. Thus, the concept of "combination preparation" or "combination medication" according to the present invention includes that the drug on the one hand and the EPO or an analogue thereof on the other hand are comprised in one common formulation or administration form, or in separate formulations or administration forms. Correspondingly, the pharmaceutical medication or preparation according to the invention may be embodied by one formulation in the form of a unit composition, or alternatively as "kit-of-part" products sharing the common purpose of being used separately, simultaneously, or sequentially. The combined use lies in the prophylaxis and/or treatment of the disease to be treated by the selected drug, such as inflammation, including inflammatory pain; related disease conditions; or other drug-specific diseases and especially cytotoxic drug-associated treatment schemes, including cancer, colon adenoma, and adenomatus polyps.

In the pharmaceutical combination preparation or medication according to one embodiment of the present invention, the drug is a selected from the group of COX-1 inhibitors and COX-2 inhibitors, including those unspecifically inhibiting both COX-1 and COX-2, those specifically inhibiting COX-1, and those specifically inhibiting COX-2.

COX-1 and/or COX-2 inhibitors are known as anti-inflammatory drugs are known to the person skilled And particularly,include drugs such as non-steroid anti-rheumatics (also known as non-steroidal anti-inflammatory drugs, NSAIDs), and Cox-2 inhibitors.

Examples for anti-inflammatory drugs include, but are not limited to Alclofenac; Alclometasone Dipropionate; Algestone Acetonide; Alpha Amylase; Amcinafal; Amcinafide; Amfenac Sodium; Amiprilose Hydrochloride; Anakinra; Anirolac; Anitrazafen; Apazone; Balsalazide Disodium; Bendazac; Benoxaprofen; Benzydamine Hydrochloride; Bromelains; Broperamole; Budesonide; Carprofen; Cicloprofen; Cintazone; Cliprofen; Clobetasol Propionate; Clobetasone Butyrate; Clopirac; Cloticasone Propionate; Cormethasone Acetate; Cortodoxone; Celecoxib; Rofecoxib (VIOXX); Etoricoxib; Valdecoxib; Parecoxib; lumiracoxib; Deflazacort; Desonide; Desoximetasone; Dexamethasone Dipropionate; Diclofenac; Diclofenac Potassium; Diclofenac Sodium; Diflorasone Diacetate; Diflumidone Sodium; Diflunisal; Difluprednate; Diftalone; Dimethyl Sulfoxide; Drocinonide; Endrysone; Enlimomab; Enolicam Sodium; Epirizole; Etodolac; Etofenamate; Felbinac; Fenamole; Fenbufen; Fenclofenac; Fenclorac; Fendosal; Fenpipalone; Fentiazac; Flazalone; Fluazacort; Flufenamic Acid; Flumizole; Flunisolide Acetate; Flunixin; Flunixin Meglumine; Fluocortin Butyl; Fluormetholone Acetate; Fluquazone; Flurbiprofen; Fluretofen; Fluticasone Propionate; Furaprofen; Furobufen; Halcinonide; Halobetasol Propionate; Halopredone Acetate; Ibufenac; Ibuprofen; Ibuprofen Aluminium; Ibuprofen Piconol; Ilonidap; Indomethacin; Indomethacin Sodium; Indoprofen; Indoxole; Intrazole; Isoflupredone Acetate; Isoxepac; Isoxicam; Ketoprofen; Lofemizole Hydrochloride; Lornoxicam; Loteprednol Etabonate; Meclofenamate Sodium; Meclofenamic Acid; Meclorisone Dibutyrate; Mefenamic Acid; Meloxicam (Mobic^{™}); Mesalamine; Meseclazone; Methylprednsisolone Suleptanate; Morniflumate; Nabumetone; Naproxen; Naproxen Sodium; Naproxol; Nimazone; Olsalazine Sodium; Orgotein; Orpanoxin; Oxaprozin; Oxyphenbutazone; Paranyline Hydrochloride; Pentosan Polysulfate Sodium; Phenbutazone Sodium Glycerate; Pirfenidone; Piroxicam; Piroxicam Cinnamate; Piroxicam Olamine; Pirprofen; Prednazate; Prifelone; Prodolic Acid; Proquazone; Proxazole; Proxazole Citrate; Rimexolone; Romazarit; Salcolex; Salnacedin; Salsalate; Salycilates; Sanguinarium Chloride; Seclazone; Sermetacin; Sudoxicam; Sulindac; Suprofen; Talmetacin; Talniflumate; Talosalate; Tebufelone; Tenidap; Tenidap Soidum; Tenoxicam; Tesicam; Tesimide; Tetrydamine; Tiopinac; Tixocortol Pivalate; Tolmetin; Tolmetin Sodium; Triclonide; Triflumidate; Zidometacin; Zomepirac Sodium; Etanercept; Lenercept; and Infliximab.. As drugs selected from COX-1 and/or COX-2 inhibitors, those selected from the nonsteroidal anti-inflammatory drugs (NSAIDs) ibuprofen, aspirin, naproxen, sulindac, piroxicam, tolmetin, diclofenac, indomethacin, fenoprofen, ketoprofen, meclofenamate are preferred, and as drugs specific for COX-2, those selected from the group consisting of celecoxib, rofecoxib, valdecoxib, parecoxib, lumiracoxib, etoricoxib, and meloxicam are preferred, respectively in view of controlling their respective adverse effects.

These and further drugs belonging to the group of COX-1 inhibitors and/or COX-2 inhibitors are known and may be revealed from common drug listings, such as "Rote Liste" (drug index of Germany, current edition, Cantor Verlag, Aulendorf, Germany), "Merck Index" (MERCK Publishing, current edition), and other similar publications.

The drug selected from the group of COX-1 and/or COX-2 inhibitors is preferably contained in the common or separate pharmaceutical formulation(s) in an amount sufficient to exert anti-phlogistic, analgetic and/or anti-pyretic effects. The drug is thus therapeutically effective to treat special disease conditions as desired, such as arthritis, especially rheumatoid arthritis, chronic polyarthritis, or osteoarthritis; arthrose; bursitis; fibromyalgia; gout; pain conditions of different origins (back pain; headache; migraine, etc.); heliobacter pylori-associated disease condititons and other infection-associated disease conditions; other inflammatory disease conditions and associated pain; or the like.

Suitable amounts of the drug selected from the group of COX-1 and/or COX-2 inhibitors per common or separate formulation or administration form containing that drug lie in the range of about 0.1 mg to about 5 g, preferably from about 5 mg to about 500 mg, more preferably from about 10 mg to about 100 mg per total amount of the drug-containing formulation. The maximum dose per day should not exceed 5 g, more preferably 3.5 g and in particular 2 g, depending on the drug to be used and the disease condition to be treated.

The other active principle of the pharmaceutical combination preparation or pharmaceutical combination medication according to the present invention is erythropoietin (EPO) or any analogue of EPO. EPO of any origin, or an EPO analogue having the ability to stimulate or increase the number of endothelial progenitor cells can be used according to the present invention, particularly recombinant human erythropoietin (rhEPO) or its analogues such as darbepoetin. The EPO analogues useful in the present invention includes derivatives or modified forms displaying EPO-like activities, in particular being likewise effective to stimulate and increase the number of endothelial progenitor cells, for example darbepoietin. Possible forms of erythropoietin are erythropoietin alpha, erythropoietin beta and others forms.

The amount of EPO or EPO analogue contained in the common or separate pharmaceutically acceptable formulation(s) again is preferably effective to control, or to reduce the risk of, acute coronary syndrome, vascular disease including coronary artery disease, myocardial infarct, thrombosis, heart insufficiency, stroke , kidney diseases, peripheral arterial disease, congestive heart failure, myocarditis, cardiomyopathy, and cerebral vascular disturbances. Suitable doses of EPO or an analogue thereof per common or separate formulation may lie in the range of 0.001 µg or more, preferably about 1 µg or more. As to the upper limit, an amount per dose of 1 mg or less and preferably 100 µg or less is suitable. As with the alternative concept of using a cytostatic drug therapy, a low dose range of EPO or an analogue thereof is used according to a preferred embodiment of COX-1 and/or COS-2 drug therapy or prophylaxis, which means a dose level which does not increase the hematocrite level. Such a low dose of EPO or the EPO analogue will be sufficient to exert its effects on endothelial progenitor cells A suitable low dose level, for example, is a range of about 0.001 µg/kg bodyweight to about 1 µg/kg bodyweight, more preferably about 0.01 µg/kg bodyweight to about 0.5 µg/kg bodyweight of EPO or an analogue thereof. The combination medication correspondingly comprises EPO or its analogue at a low dose, e.g. at an amount of lower than 100 µg, preferably lower than 50 µg, particularly lower than 10 µg, with respect to the total amount of the common or separate EPO or EPO analogue containing formulation (1000 I.E. typically corresponds to about 8.3-8.4 µg active agent EPO). The treatment scheme of such a low dose use of EPO or its analogue beneficially includes administration of one dose per day, up to one dose per week. In particular, one or two low doses per week are sufficiently effective to activate, or increase the number of endothelial progenitor cells.

The common or separate administration form, or the respective formulation(s) containing the drug and EPO or its analogue, respectively, can be suitably used as desired and as appropriate. Suitable formulation(s) or administration forms include, but are not limited to: oral; parenteral; by injection through bolus, intravenously, intramuscularly, and subcutaneously; by infusion; transdermally; nasally; by inhalation; or any other suitable administration forms.

A further suitable constituent of the pharmaceutical combination preparation or medication of the present invention is a pharmaceutically acceptable carrier or excipient. Any conventional and known carrier, excipient or additive used for either one of the above-mentioned active agents (drugs from the group of COX-1 and COX-2 inhibitors; and EPO or analogue thereof) can be used. When the combined active agents shall be included in a common administration form, carrier or excipient and other constituents of the formulation should be compatible with both active agents. If separate formulations are preferred for simultaneous or sequential use of both, the drug and EPO or its analogue, the carriers, excipients or other constituents of the formulations need to be compatible only for each active agent can be selected. In the case of separate but combined use, also the optimal administration forms for the respective active drugs is selectable, for example orally or topically for the NSAID drug, and by injection for EPO or its analogue, although the present invention is not limited thereto. Depending on the desired formulation(s), a pharmaceutical combination preparation or medication being solid, semi-solid, gelatinous or hydrogel-like, a liquid, a suspension, an emulsion, a powder, an ointment, a cream, an oil, a tablet, a pill, a capsule, a suppository, a sustainedrelease dosage form and the like can be used.

For example, solid or semi-solid oral forms may contain, besides the active agents, diluents such as cellulose, starch, dextrose, saccharose, lactose; lubricants such magnesium or calcium stearate, stearic acid, talc, silica, polyethylene glycols; binding agents such as starch, methyl cellulose, caboxymethyl cellulose or other cellulose types, gelatine and polyvinyl pyrrolidone; fillers such as microcristalline cellulose, metal oxide fine particles and the like; disintegrating agents such as alginic acid, algenates, sodium starch glycolate; wetting agents such as polysorbates, lauryl sulfates, lecithin; coloring agents; sweeteners; aroma substances; and the like. Suitable manufacturing processes for preparing such oral forms may, for example, include mixing, granulating, coating such as sugarcoating or other film-coating, tabletting, and the like. Liquid forms for oral administration may be, for example, sirups, suspensions and emulsions with appropriate liquid medium and optionally a carrier such as mannitol, sorbitol, saccharose; alcohols such as ethanol; glycerine; polyvinyl alcohol; cellulose types; and the like.

The solutions or suspensions for intramuscular or subcutaneous injections may contain, besides the active agents, a pharmaceutically acceptable carrier such as sterile water; oils; glycols such as propylene glycol; oleate; or the like. Solutions for bolus or i.v. injections or infusions may contain as carrier, for example, sterile water, preferably sterile aqueous isotonic saline solutions, optionally mixed with stabilizers and/or other additives, such as buffering agents such as phosphates; sodium chloride; polysorbates such as polysorbate 20 or polysorbate 80; amino acids such as glycine; urea; and the like.

Suppositories may contain, besides the active agents, polyethylene glycol; polysorbates; lecithin; and other suitable carriers.

Ointments, creams, oils and similar other topical administration forms may include, for example, plant oils such as almond oil, olive oil, peanut oil, ricinus oil or the like; plant extracts, etheric oils, vitamin oils, fatty stubstances and fatty-like substances, lipoids, phospholipids, hydrocarbons such as paraffins, vaseline, lanoline, wax and the like; detergents; adjuvant agents for skin, such as lecithin, wool grease alcohols, carotene, skin nutritions, and the like; perfumes; cosmetic substances; alcohols; glycerine; glycol; urea; talc; preservatives; coloring agents such as titania or zinc white; antioxidants; etc.. As base substance, water is generally used, obtaining typically 0/W- oder W/Oemulsions by addition of emulsifiers such as fatty alcohols sulfates, alkali soaps, lecithin, triethanolamine and the like.

Suitable embodiments of pharmaceutical combination preparations or pharmaceutical medications according to the present invention may include, but are not limited to:
1) administration of rhEPO or an EPO analogue in a common combined administration form, with a low dose of the EPO active agent combined with a drug from the group of COX-1 or COX-2 inhibitors at typical dose, formulated as an i.v. or s.c. injection, or optionally orally at a suitable timing such as once a day;
2) administration of active agents separately and at suitable timings - preferably administering rhEPO or EPO analogue by i.v. or s.c. injection once or twice a week or more suitable in a new formulation monthly, and simultaneously or subsequently administering the drug selected from the group of COX-1 and/or COX-2 inhibitors orally or topically or by infusion once, twice or three times a day; and
3) combining previously separated formulations, respectively containing the corresponding active agent with a suitable carrier or excipient, to form a common administration form for immediate simultaneous use, for example by bolus i.v. or s.c. injection or by infusion.

The present invention is described in more detail in the following by referring to illustrative, but non-limitive examples:

### Example 1:

A pharmaceutical combination preparation according to the invention can be formulated as follows.
COX-1/COX-2-drug formulation:

A gel for topical administration against rheumatic disease conditions is obtained by admixing the following constituents:

| | |
|---|---|
| ibuprofen | 5.0 g |
| isopropyl alcohol | 70.0 g |
| isopropyl myristate | 2.5 g |
| polysorbate 80 | 0.5 g |
| lavender oil | 0.5 g |
| purified water | 22.5 g ad 100.0 g |

As a separate formulation, an EPO containing solution is obtained by mixing the following constituents:

| | |
|---|---|
| rhEPO alpha | 20,000 I.E. (corresponding to 168 µg) |
| polysorbate 20 | 1 mg glycine |
| 2 mg NaCl | 10 mg |
| Na₂HPO₄ * 2H₂O | 10 mg |
| Na₂HPO₄ | 5 mg |
| purified water | ad 10 ml |

The obtained aqueous solution is sterilized and sealed within ampoules at a volume of 1 ml solution per ampoule. Each ampoule can be used for subcutaneous injection to a patient applying the anti-rheumatic gel described above.

### Example 2:

Hard tablets containing celecoxib as a COX-2 specific drug can be manufactured as follows:

| | |
|---|---|
| celecoxib | 100 g |
| lactose | 300 g |
| corn starch | 250 g |
| talc powder | 20 g |
| magnesium sterate | 5g |

The components are suspended in 50 ml warm water, homogenously mixed and processed into 1,000 tablets. Each tablet obtained is suitable for oral administration, for example to treat chronic arthritis in a patient.

Separately, an injectable EPO analogue can be prepared as follows: monopegylated erythropoietin analogues can be prepared as described (see e.g. D.L. Long et al., Ex. Hematol. 2006, 34, pp. 697-704). The maleimide-PEG attached to EPO cystein analogues can be formulated by admixing 100 pg of the EPO analogue with 0.9% aqueous NaCl to obtain 10 ml sterile solution. The obtained solution can be distributed to 10 syringe doses for i.v. injections to the patient undergoing the above mentioned celecoxib treatment against chronic arthritis.

### Example 3:

An administration scheme according to the present invention to treat a patient having arthritis symptoms can be designed, by first administering to a patient in need of anti-phlogistic treatment 1,000 I.E. (8.3 µg) rhEPO alpha through a "ready-touse" syringe injection (NeoRecormon^{®} sold by Roche) by a s.c. injection two weeks prior to starting anti-phlogistic treatment. The same s.c. injection is repeated one week prior to starting anti-phlogistic treatment. Then, the patient can start anti-phlogistic treatment by orally taking a selective COX-2 inhibitor (rofecoxib, Vioxx^{®}, sold by Merck) at a dose of one 25 mg tablet per day. Parallel to the daily oral use of the corecoxib tablet but in separate administration forms, the s.c. injection of rhEPO (NeoRecormon^{®} at 1,000 I.E.) is continued once per week. This administration scheme can be continued as long as the patient is in need of anti-phlogistic treatment.

### Example 4:

As separate formulations there are provided:
(1) EPO containing injection solution obtained by mixing the following constituents:

| | |
|---|---|
| rhEPO alpha | 5,000 I.E. (corresponding to 42 µg) |
| polysorbate 80 | 0.5 mg |
| glycine | 1 mg |
| leucin | 1 mg |
| threonin | 1 mg |
| urea | 0.2 mg |
| polyvinylpyrrolidone | 0.5 mg |
| NaCl | 10 mg |
| Na₂HPO₄ * 2H₂O | 10 mg |
| Na₂HPO₄ | 5 mg |
| purified water | ad 10 ml |

The obtained aqueous solution is sterilized and sealed within ampoules at a volume of 1 ml solution per ampoule. Each ampoule is suitable for subcutaneous injection.
(2) Paclicatexel (Taxol) infusion solution containing, as a 5 ml concentrate solution:

| | |
|---|---|
| paclitaxel | 30 mg |
| ethylene glykol | 5 mg |
| CaCl₂ | 3 mg |
| Ethanol | 2.5 ml |
| pure water | ad 5.0 ml |

A cancer patient receives Taxol infusion, prepared from the 5 ml concentrate solution according to (2), a cancer treatment scheme. Parallel with Taxol infusion, the patient receives a 1 ml apoule dosage by subcutaneous injection according to (1). Generation of endothelial progenitor cells (EPCs) are determined as described (Bahlmann et al., Blood 103, 921-926 (2004). Under further monitoring of EPCs, 1 ml doses of EPO per week by subcutaneous injection according to (1), as well as parallel Taxol infusion administrations are continued. EPO administration is continued for 12 weeks.

## Claims

1. A pharmaceutical combination preparation comprising as active agents, in a common or in separate pharmaceutically acceptable formulation(s):
a drug selected from the group of COX-1 and/or COX-2 inhibitors; and
erythropoietin (EPO) or an analogue of EPO.

2. Pharmaceutical combination medication for separate, simultaneous or sequential use in prophylaxis and/or treatment of inflammation or inflammation-associated pain, said combined medication comprising as active agents, in a common or in separate pharmaceutically acceptable formulation(s): a drug selected from the group of COX-1 and/or COX-2 inhibitors in an amount therapeutically effective to treat inflammation or inflammation-associated pain; and erythropoietin (EPO) or an analogue of EPO in an amount effective to stimulate or to increase the number of endothelial progenitor cells.

3. Pharmaceutical combination preparation or medication according to claim 1 or 2, wherein the EPO or the EPO analogue is contained in the common or separate pharmaceutically acceptable formulation(s) in an amount effective to control, or to reduce the risk of cardiovascular complications, acute coronary syndrome, vascular disease, myocardial infarct, thrombosis, heart insufficiency and stroke.

4. The pharmaceutical combination preparation according to any one of claims 1 to 3, wherein said drug is selected from COX-2 specific inhibitors by being selected from the group consisting of celecoxib, rofecoxib, valdecoxib, parecoxib, lumiracoxib, etoricoxib, and meloxicam.

5. The pharmaceutical combination preparation according to any one of claims 1 to 3, wherein said drugs selected from COX-1 and/or COX-2 inhibitors are non-steroidal antiinflammatory drugs (NSAIDs) selected from ibuprofen, aspirin, naproxen, sulindac, piroxicam, tolmetin, diclofenac, indomethacin, fenoprofen, ketoprofen, meclofenamate, optionally prostaglandins such as misoprostol.

6. The pharmaceutical combination preparation or medication according to any one of the preceding claims, wherein the amount of EPO or EPO analogue is lower than about 10 pg, with respect to the total amount of the common or separate EPO or EPO analogue containing formulation.

7. A method for treatment and/or prophylaxis of inflammation, in particular arthritis, the method comprising the steps of:
administering to a patient in need of such treatment or prophylaxis, a drug selected from the group of COX-1 and/or COX-2 inhibitors; and
a step of administering to said patient, in an administration separately, simultaneously or sequentially with said drug, erythropoietin (EPO) or an EPO analogue.

8. A method for controlling, or for reducing the risk of adverse effects associated with the therapeutic administration of a drug selected from the group of COX-1 and/or COX-2 inhibitors, comprising the steps of:
administering a drug selected from the group of COX-1 and/or COX-2 inhibitors to a patient in need of such therapeutic treatment; and
administering, in an administration separately, simultaneously or sequentially with said COX-1 and/or COX-2 drug, erythropoietin (EPO) or an EPO analogue to said patient.

9. The method according to claim 8, wherein said step of administering EPO or the EPO analogue is effective to control, or to reduce the risk of, acute coronary syndrome, vascular disease, myocardial infarct, thrombosis, heart insufficiency and stroke.

10. The method according to any one of claims 7 to 9, wherein said EPO or EPO analogue is administered in an amount lower than about 1.0 µg/kg bodyweight.

11. Pharmaceutical combination medication for separate, simultaneous or sequential use in prophylaxis and/or treatment of primary diseases treated by cytotoxic drug therapy, including cancer, colon adenoma, and adenomatus polyps;
said combined medication comprising as active agents, in a common or in separate pharmaceutically acceptable formulation(s):
a cytotoxic drug selected from the group consisting of cytostatic agents; alkylating
agents; antimetabilites; sulfonamides; antibiotics; antomycotics; antibodies and other cytotoxic or cardiotoxic drugs, respectively in an amount therapeutically effective to treat said primary disease;
and erythropoietin (EPO) or analogues of EPO in an amount effective to stimulate or to increase the number of endothelial progenitor cells.

12. The pharmaceutical combination medication according to claim 11, used for the prophylaxis and/or treatment of secondary kidney diseases, peripheral arterial disease, congestive heart failure, myocarditis, cardiomyopathy, and cerebral vascular disturbances, respectively caused by the adminsitration of said cytotoxic drug in said primary disease.

13. The pharmaceutical combination medication according to claim 11 or 12, wherein the amount of EPO or EPO analogue is lower than about 10 µg, with respect to the total amount of the common or separate EPO or EPO analogue containing formulation.

14. A method for adjuvant treatment and/or prophylaxis in primary diseases treated by cytotoxic drug therapy, including colon adenoma, cancerand adenomatus polyps,
the method comprising the steps of:
administering to a patient in need of such treatment or prophylaxis, a cytotoxic drug selected from the group consisting of cytostatic agents; anthracyclines; alkylating agents; angiogenese- and neoangiogenese-inhibitors; antimetabilites; sulfonamides; antibiotics; antomycotics; antipsychotrics; photosensitizers; catecholamines and monoclonal antibodies, respectively in an amount therapeutically effective to treat saidprimary disease;
and
administering, in an administration separately, simultaneously or sequentially with said cytotoxic drug, erythropoietin (EPO) or an EPO analogue to said patient in an amount effective to stimulate or to increase the number of endothelial progenitor cells,

15. The method according to claim 14, used for the prophylaxis and/or treatment of secondary kidney diseases, peripheral arterial disease, congestive heart failure, myocarditis, cardiomyopathy, and cerebral vascular disturbances, respectively caused by the adminsitration of said cytotoxic or cardiotoxic drug in the treated primary disease.

16. The method according to claim 14 or 15, wherein said EPO or EPO analogue is administered in an amount lower than about 1.0 µg/kg bodyweight.
